# EUROPEAN PATENT APPLICATION

(11) **EP 3 031 463 A1**
(43) Date of publication of application: **15.06.2016**
(21) Application number: 15199138.7
(22) Date of filing: 10.12.2015
(51) Int. Cl.: A61K 35/14, A61K 35/28, A61P 37/00

(54) **METHOD OF PREPARING INJECTION SOLUTION**

(30) Priority: 13.12.2014 US 201462091522 P
(71) Applicant: Stembios Technologies, Inc., Monterey Park CA 91754 (US)
(72) Inventor: Wang, James, Monterey Park, CA California 91754 (US)
(74) Representative: Wittmann, Günther

(57) **Abstract**

A method of preparing an injection solution includes: (1) storing a mixture of a blood sample and a divalent cation chelating agent at a temperature between 2 and 12 degrees Celsius for a period of from 3 hours to 72 hours so as to have the mixture with two or more separate layers, wherein a liquid in one of the separate layers contains small somatic stem cells such as SB cells; (2) collecting the liquid from the mixture; and (3) after collecting the liquid from the mixture, mixing the liquid with a solution free from Ca²⁺ into the injection solution. The injection solution can be used for an autoimmune disease, such as ankylosing spondylitis, rheumatoid arthritis, or Grover's disease.

## Description

### BACKGROUND OF THE DISCLOSURE

This application claims priority to U.S. provisional application No. 62/091,522, filed on Dec. 13, 2014, which is incorporated herein by reference in its entirety.

### Field of the Disclosure

The disclosure relates to a method of preparing a solution containing cells, and more particularly, to a method of preparing an injection solution containing (somatic) stem cells.

### Brief Description of the Related Art

Stem cells have the ability to self-renew to generate more stem cells and also to become almost any type of specialized cells. Stem cell research is useful for learning about human development and is one of the most fascinating areas of contemporary biology. Therefore, stem cells offer exciting promise for future medical science.

### SUMMARY OF THE DISCLOSURE

An exemplary embodiment of the present disclosure provides a method of forming or preparing a stem-cell-containing solution (or called a stem-cell mixture), which may be used as an injection liquid or solution for treating an autoimmune disease such as ankylosing spondylitis, rheumatoid arthritis, or Grover's disease. The method includes: (1) storing a first mixture of a blood sample and a divalent cation chelating agent (e.g., EDTA) at a temperature between 2 and 12 degrees Celsius (°C), and more preferably between 2 and 7 °C, for a period of from 3 hours to 72 hours, from 3 hours to 6 hours, from 6 hours to 72 hours, from 6 hours to 48 hours, from 16 hours to 72 hours, from 16 hours to 48 hours, from 36 hours to 60 hours, or from 48 hours to 72 hours, so as to have the first mixture with two or more separate layers (e.g., including an upper layer and a lower layer), wherein a liquid (e.g., a supernatant liquid) in one of the separate layers (e.g., the upper layer or the topmost one of the separate layers) contains platelets (which, for example, are less than 6 micrometers in size), one or more types of somatic stem cells (such as CD349(+) somatic stem cells and Lgr5(+) somatic stem cells), the divalent cation chelating agent, and growth factors; (2) collecting the liquid from the first mixture; and (3) after collecting the liquid from the first mixture, mixing the liquid with a medium or solution free from Ca²⁺ into a second mixture. The medium or solution free from Ca²⁺ may be further free from any other divalent ions, including Mg²⁺. For example, the medium or solution free from Ca²⁺ is a salt solution, such as normal saline. The second mixture may be the stem-cell-containing solution. Alternatively, the method may further include filtering the second mixture so as to prepare or form the stem-cell-containing solution.

Another exemplary embodiment of the present disclosure provides a method of forming or preparing a stem-cell-containing solution (or called a stem-cell mixture), which may be used as an injection liquid or solution for treating an autoimmune disease such as ankylosing spondylitis, rheumatoid arthritis, or Grover's disease. The method includes: (1) incubating a blood sample with a divalent cation chelating agent (e.g., EDTA) at a temperature between 2 and 12 °C, and more preferably between 2 and 7 °C, for a time period, such as between 3 hours and 72 hours, and more preferably between 3 hours and 6 hours, between 6 hours and 72 hours, between 6 hours and 48 hours, between 16 hours and 72 hours, between 16 hours and 48 hours, between 36 hours and 60 hours, or between 48 hours and 72 hours, so that the blood sample is formed with multiple separate layers (e.g., including an upper layer and a lower layer); and (2) collecting one of the separate layers (e.g., the upper layer) into the stem-cell-containing solution, wherein said one of the separate layers contains platelets (which, for example, are less than 6 micrometers in size), somatic stem cells (which contain CD349(+) somatic stem cells and Lgr5(+) somatic stem cells), the divalent cation chelating agent, and growth factors. Alternatively, the method may further include mixing said one of the separate layers with a medium or solution free from Ca²⁺ into the stem-cell-containing solution. The medium or solution free from Ca²⁺ may be further free from any other divalent ions, including Mg²⁺. For example, the medium or solution free from Ca²⁺ is a salt solution, such as normal saline.

In the above-mentioned methods, the somatic stem cells, for example, are between 1 and 6 micrometers in size, and more preferably greater than 1 or 2 micrometers and less than 6 micrometers in size. The somatic stem cells may also contain CD66e(+) somatic stem cells, i.e., blastomere-like stem cells (BLSCs), which may be greater than 2 micrometers and less than 6 micrometers in size. The CD349(+) somatic stem cells are also CD9(+) and, for example, are greater than 2 micrometers and less than 6 micrometers in size. The Lgr5(+) somatic stem cells are also Oct4(+) and Nanog(+), as well as CD133(-), CD66e(-), Sox2(-), CD4(-), CD8(-), CD9(-), CD10(-), CD11(-), CD16(-), CD17(-), CD18(-), CD19(-), CD20(-), CD21(-), CD31(-), CD42(-), CD63(-), CD34(-), Lin(-), CD38(-), CD90(-), CD45(-), and CD349(-). The Lgr5(+) somatic stem cells, for example, are greater than 2 micrometers and less than 6 micrometers in size. The percentage of the Lgr5(+) somatic stem cells in cells greater than 2 micrometers and less than 6 micrometers in size in the stem-cell-containing solution is greater than 4% or 5%, and more preferably between 4.5% and 10%. The percentage of the CD349(+) somatic stem cells in cells greater than 2 micrometers and less than 6 micrometers in size in the stem-cell-containing solution is greater than 4% or 5%, and more preferably between 4.5% and 10%. Less than 1% of cells in the stem-cell-containing solution may express CD 133. The percentage of white blood cells in cells greater than 2 micrometers and less than 20 micrometers in size in the stem-cell-containing solution is less than 2%, and more preferably less than 1%, 0.5% or 0.1%. The percentage of red blood cells in cells greater than 2 micrometers and less than 10 micrometers in size in the stem-cell-containing solution is less than 3%, and more preferably less than 2%, 1% or 0.5%. The blood sample may be derived from the peripheral blood of a subject at a time point when a stem-cell mobilization agent (e.g., a fucoidan-containing compound) is administered to the subject a time period, e.g., between 1 hour and 6 hours, later. The subject is a human or an animal. The stem-cell-containing solution may contain a salt, such as sodium chloride (NaCl).

Another exemplary embodiment of the present disclosure provides an injection liquid or solution (i.e., the stem-cell-containing solution) for treating an autoimmune disease such as ankylosing spondylitis, rheumatoid arthritis, or Grover's disease. The injection liquid or solution contains CD349(+) somatic stem cells (SB-1 cells) that are greater than 2 micrometers and less than 6 micrometers in size, Lgr5(+) somatic stem cells (SB-2 cells) that are greater than 2 micrometers and less than 6 micrometers in size, and CD66e(+) somatic stem cells (blastomere-like stem cells) that are greater than 2 micrometers and less than 6 micrometers in size. The injection liquid or solution may further contain a divalent cation chelating agent (e.g., EDTA), growth factors, and/or a salt (e.g., sodium chloride). The percentage of the Lgr5(+) somatic stem cells in cells greater than 2 micrometers and less than 6 micrometers in size in the injection liquid or solution is greater than 4% or 5%, and more preferably between 4.5% and 10%. The percentage of the CD349(+) somatic stem cells in cells greater than 2 micrometers and less than 6 micrometers in size in the injection liquid or solution is greater than 4% or 5%, and more preferably between 4.5% and 10%. The percentage of the CD66e(+) somatic stem cells in cells greater than 2 micrometers and less than 6 micrometers in size in the injection liquid or solution may be less than 6%, and more preferably less than 5% or 4.5%. Less than 1% of cells in the injection liquid or solution express CD133. The percentage of white blood cells in cells greater than 2 micrometers and less than 20 micrometers in size in the injection liquid or solution may be less than 2%, and more preferably less than 1%, 0.5% or 0.1%. The percentage of red blood cells in cells greater than 2 micrometers and less than 10 micrometers in size in the injection liquid or solution may be less than 3%, and more preferably less than 2%, 1% or 0.5%.

Another exemplary embodiment of the present disclosure provides a method for treating an autoimmune disease, such as ankylosing spondylitis, rheumatoid arthritis, or Grover's disease, in a human subject. The method includes administering to the human subject in need thereof an effective amount of the injection liquid or solution so as to inhibit, cure or treat the autoimmune disease in the human subject. The Lgr5(+) somatic stem cells, the CD 349(+) somatic stem cells, and the CD66e(+) somatic stem cells in the injection liquid or solution, for example, are autologous to the human subject. The step of administering to the human subject the injection liquid or solution may include intravenously injecting the injection liquid or solution mixed with a medium or solution free from Ca²⁺ into the human subject. The medium or solution free from Ca²⁺ may be further free from any other divalent ions, including Mg²⁺. For example, the medium or solution free from Ca²⁺ is a salt solution, such as normal saline.

These, as well as other steps, features, benefits, and advantages of the present disclosure, will now become clear from a review of the following detailed description of illustrative embodiments, the accompanying drawings, and the claims.

### BRIEF DESCRIPTION OF THE DRAWINGS

Aspects of the disclosure may be more fully understood from the following description when read together with the accompanying drawings, which are to be regarded as illustrative in nature, and not as limiting. In the drawings:
Fig. 1 shows a flow chart illustrating a treatment or therapy for an autoimmune disease according to an embodiment of the present disclosure;
Fig. 2 shows the content/ingredient information of a brown algae supplement;
Fig. 3 shows a flow chart illustrating a method of forming or preparing a stem-cell-containing solution according to an embodiment of the present disclosure;
Fig. 4 shows stem-cell data obtained from six human subjects before and after orally ingesting a brown algae supplement;
Figs. 5A and 5B show flow cytometry data obtained from a human subject before and after a course of injections with granulocyte-colony stimulating factor (GCSF or G-CSF);
Fig. 6A is a forward scattering coefficient (FSC) versus side scattering coefficient (SSC) flow cytometry dot plot;
Fig. 6B is a fluorescence histogram from flow cytometry analysis;
Fig. 6C is a FSC versus SSC flow cytometry dot plot;
Fig. 7A is a FSC versus SSC flow cytometry dot plot; and
Figs. 7B-7G are fluorescence histograms from flow cytometry analysis.

While certain embodiments are depicted in the drawings, one skilled in the art will appreciate that the embodiments depicted are illustrative and that variations of those shown, as well as other embodiments described herein, may be envisioned and practiced within the scope of the present disclosure.

### DETAILED DESCRIPTION OF THE INVENTION

Illustrative embodiments are now described. Other embodiments may be used in addition or instead. Details that may be apparent or unnecessary may be omitted to save space or for a more effective presentation. Conversely, some embodiments may be practiced without all of the details that are disclosed.

Before describing embodiments of the present invention, a definition or description has been included for these various terms. These definitions or descriptions are provided to assist in teaching a general understanding of the present invention.

### Definition of size (Z) of a cell:

The size (Z) of a cell such as a stem cell or a biological cell, mentioned in all following paragraphs, of the present disclosure may be, but not limited to, described or defined as (1) the conventional definition of the size or representative length of a cell in the field of cell biology or the field of stem cells, (2) the diameter of a cell especially when the cell is substantially spherical, (3) the length of the major axis of a cell especially when the cell is substantially ellipsoidal, (4) the width of a cell when the shape of the cell has an approximate shape of a square, (5) the length of a cell when the shape of the cell has an approximate shape of a rectangle, or (6) the greatest cross-sectional or transverse dimension of a cell. The size (Z), either the diameter, length, width, or greatest cross-sectional or transverse dimension, can be, but not limited to, determined or measured, for example, using an image of the cell obtained from an optical microscope or from an electron microscope (e.g., scanning electron microscope (SEM)), or using data (e.g., two-dimensional dot, contour or density plot) of the cell obtained from a flow cytometer. The image of the cell obtained from the optical microscope or electron microscope may be a two-dimensional (2D) cross section or three-dimensional (3D) structure of the cell. As an example, the size (Z) of the cell may be obtained by, e.g., measuring the greatest cross-sectional or transverse dimension of the cell in a 2D cross-sectional image obtained from an optical microscope or an electron microscope (e.g., SEM).

### Description of stem cells:

Somatic stem cells (also called adult stem cells) can be found in an organ or tissue such as bone marrow, fat or peripheral blood and possess the same basic characteristics of all stem cells. A somatic stem cell is an unspecialized or undifferentiated cell, which is capable of differentiation into specialized cell types. In the present disclosure, somatic stem cells are not embryonic stem cells; in other words, the somatic stem cells are not derived, sourced or harvested from embryos or fetal tissue.

There are various types of somatic stem cells, including totipotent stem cells, pluripotent stem cells, multipotent stem cells, and progenitor stem cells (also called unipotent stem cells). Blastomere-like stem cells (BLSCs) are totipotent or pluripotent somatic stem cells. Very small embryonic-like stem cells (VSELs) are pluripotent somatic stem cells. SB-1 cells and SB-2 cells are pluripotent or multipotent somatic stem cells. Mesenchymal stem cells (MSCs), hematopoietic stem cells (HSCs), multipotent adult progenitor cells (MAPCs), bone marrow derived multipotent stem cells (BMSCs), and multipotent adult stem cells (MASCs) are multipotent somatic stem cells. Neural stem cells, retina stem cells, olfactory bulbs stem cells, epidermal stem cells, muscle stem cells, intestine stem cells, pancreatic stem cells, heart stem cells, liver stem cells, kidney stem cells, endothelial stem cells, adipocyte or adipose-derived stem cells, marrow-isolated adult multilineage inducible (MIAMI) cells, pre-mesenchymal stem cells (pre-MSCs), mesenchymal progenitor cells, hematopoietic progenitor cells (HPCs), multipotent progenitor cells (MPPs), lineage-restricted progenitor cells (LRPs), common myeloid progenitor cells (CMPs), and common lymphocyte progenitor cells (CLPs) are progenitor stem cells.

In the following paragraphs the sign "+" following a cell (surface) marker means cells (e.g., stem cells) can express the cell (surface) marker; in the other term, the cell (surface) marker existing in the cell surfaces of the cells may be detected by performing a flow cytometry using a (marker-specific) antibody. And, the sign "-" following a cell (surface) marker means cells (e.g., stem cells) do not express the cell (surface) marker; in the other term, the cell (surface) marker, not existing in the cell surfaces of the cells, may not be detected by performing a flow cytometry using a (marker-specific) antibody. The positive sign "+" is a positive expression of a cell (surface) marker for a cell such as a stem cell, and the negative sign "-" is a negative expression of a cell (surface) marker for a cell such as a stem cell.

A SB-1 cell, which may be a pluripotent or multipotent somatic stem cell having a nucleus, is a CD349(+) cell. The CD349(+) somatic stem cell, i.e., the SB-1 cell, may be also CD9(+), Oct4(+), and Nanog(+), as well as CD133(-), CD90(-), CD34(-), and Sox2(-). The CD349(+) somatic stem cell is smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers, or between 1.0 and 4.0 micrometers, in size (as defined by the above-mentioned size (Z) of a cell). Preferably, the SB-1 cell is greater than 2 micrometers and less than 6 micrometers in size. The SB-1 cell can express the cell (surface) marker CD349 and can be characterized by CD349(+).

A SB-2 cell, which may be a pluripotent or multipotent somatic stem cell having a nucleus, is a Lgr5(+) cell. The Lgr5(+) somatic stem cell, i.e., the SB-2 cell, may be also Oct4(+) and Nanog(+), as well as CD133(-), CD66e(-), CD4(-), CD8(-), CD9(-), CD10(-), CD11(-), CD16(-), CD17(-), CD18(-), CD19(-), CD20(-), CD21(-), CD31(-), CD42(-), CD63(-), CD34(-), Lin(-), CD38(-), CD90(-), CD45(-), CD349(-), and Sox2(-). The Lgr5(+) somatic stem cell is smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Preferably, the SB-2 cell is greater than 2 micrometers and less than 6 micrometers in size. The SB-2 cell can express the cell (surface) marker Lgr5 and can be characterized by Lgr5(+). In the present disclosure, SB cells contain CD349(+) somatic stem cells (SB-1 cells) and Lgr5(+) somatic stem cells (SB-2 cells).

A blastomere-like stem cell (BLSC), which may be a totipotent or pluripotent somatic stem cell having a nucleus, is a CD66e(+) cell. The CD66e(+) somatic stem cell, i.e., the BLSC, may be smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Preferably, the BLSC is greater than 2 micrometers and less than 6 micrometers in size. The BLSC can express the cell (surface) marker CD66e and can be characterized by CD66e(+).

Very small embryonic-like stem cells (VSELs), which may be pluripotent somatic stem cells each having a nucleus, contain CD133(+) somatic stem cells and CD34(+) somatic stem cells. The CD133(+) somatic stem cells and the CD34(+) somatic stem cells, i.e., VSELs, may be also CD45(-) and Lin(-). The CD133(+) somatic stem cells and the CD34(+) somatic stem cells may be smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Preferably, the CD133(+) somatic stem cells and the CD34(+) somatic stem cells are greater than 2 micrometers and less than 6 micrometers in size. In the case of the CD133(+), CD45(-), Lin(-) somatic stem cell, the VSEL can express the cell (surface) marker CD133 and lacks expression of the two cell (surface) markers CD45 and Lin; in other words, the VSEL can be characterized by CD133(+), CD45(-), and Lin(-). In the case of the CD34(+), CD45(-), Lin(-) somatic stem cell, the VSEL can express the cell (surface) marker CD34 and lacks expression of the two cell (surface) markers CD45 and Lin; in other words, the VSEL can be characterized by CD34(+), CD45(-), Lin(-).

A mesenchymal stem cell (MSC), which may be a multipotent somatic stem cell having a nucleus, may be a CD13(+), CD29(+), CD44(+), CD73(+), CD90(+) or CD105(+) somatic stem cell. The MSC may express one or more of the cell (surface) markers CD13, CD29, CD44, CD73, CD90 and CD105 and may be characterized by one or more of CD13(+), CD29(+), CD44(+), CD73(+), CD90(+) and CD105(+). Mesenchymal stem cells (MSCs) are very heterogeneous populations which mean to have various sizes and shapes. Some types of MSCs may be smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). The other types of MSCs may be greater than 6, 7 or 10 micrometers in size (as defined by the above-mentioned size (Z) of a cell).

Hematopoietic stem cells (HSCs), which may be multipotent somatic stem cells each having a nucleus, contain CD34(+), cKit(-), CD38(-), Lin(-) cells and CD150(+), CD244(-), CD48(-) cells. The CD34(+), cKit(-), CD38(-), Lin(-) somatic stem cells and the CD150(+), CD244(-), CD48(-) somatic stem cells, i.e., HSCs, may be smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). In the case of the CD34(+), cKit(-), CD38(-), Lin(-) somatic stem cell, the HSC can express the cell (surface) marker CD34 and lacks expression of the cell (surface) markers cKit, CD38 and Lin; in other words, the HSC can be characterized by CD34(+), cKit(-), Lin(-) and CD38(-). In the case of the CD150(+), CD244(-), CD48(-) somatic stem cell, the HSC can express the cell (surface) marker CD150 and lacks expression of the two cell (surface) markers CD244 and CD48; in other words, the HSC can be characterized by CD150(+), CD244(-) and CD48(-).

### Description of actions (X):

The following items are examples of the actions (X), which may be previously evaluated effective in increasing the number of cells for a type or selected types of stem cells in vivo in a subject such as a human body or entity or a non-human body or entity. The non-human body or entity may be an animal body or entity. The actions (X) include:
1. Taking drugs such as synthetic drugs or drugs including extractions from nature;
2. Taking herbs or Chinese or herbal medicines, such as Cordyceps sinensis, ginseng, Lycium Chinense Mill, Ganoderma lucidum (lingzhi), Taiwanofungus camphoratus, and/or Brazil mushroom;
3. Taking nutrients or dietary supplements, such as nutrition pills or powder, including the following materials or elements: vitamins (Vitamin A, B, B complex, B₁₂, D, D₃, E, etc.), macro and/or trace minerals (e.g., calcium, sodium, potassium, fluorine, bromine, chromium, iodine, silicon, selenium, beryllium, lithium, cobalt, vanadium and/or nickel), polysaccharides, high molecular weight fucose-containing glycoproteins, seaweed (including green algae, blue-green algae, brown algae, and etc.), fucose, fucoidan that is a major component of brown algae, oligo fucoidan, algae, brown algae containing fucoidan (for example, brown algae grown and produced in Okinawa, Japan), Japanese Mozuku, green algae, blue-green algae (or blue algae), brown algae (including mozuku, kelp, undaria, sargassum fusiforme, pinnatifida, and etc.), phytochemical (e.g., isoflavones or phytoestrogen), lycopene, epigallocatechin gallate (EGCG), green tea essence, gluconutrients (e.g., Xylose, Galactose, Glucose, Mannose N-acetylglucosamine, N-acetylgalaetosanmine, or N-acetylneuraminic acid), fish oil, China toona (toona sinensis), and/or nutrients extracted from plant, leaf, fruit, vegetable, fish, seaweed, or algae;
4. Practicing a vegetarian dietary;
5. Taking or eating healthy food or organic food;
6. Taking alternative (non-traditional) medicine;
7. Being subjected to alternative therapy or treatment such as the Gerson therapy or the Breuss cancer cure;
8. Being subjected to acupuncture;
9. Being subjected to massage such as foot massage;
10. Exercising such as walking, jogging, dancing, gymnastics, Yoga, aerobic exercise, and/or Taijiquan (Chinese shadow exercise);
11. Sleeping (for purpose of measuring the quality of sleep);
12. Meditating;
13. Exercising a health improvement program or a disease curing program designed by an individual, a health professional, or a medical doctor;
14. Taking a certain nutrient for improving health of a certain organ in a body, for example, taking lycopene to improve the health of prostate;
15. Taking a rehabilitation program to heal the injury, or to heal the wounds caused by surgery, or to cure a disease;
16. Taking a medicinal liquor (or called medicinal wine, medicated liquor or medicated wine) made from, e.g., immersing one Chinese medicine or multiple Chinese medicines in liquor or wine for a period of time, such as ginseng wine made from immersing ginseng in a high alcohol concentration rice wine for a month;
17. Taking one or more drugs approved by a government department or authority, such as U.S. food and drug administration (U.S. FDA), for curing a specific disease (e.g., a type of cancer, skin disease, kidney disease and/or so on);
18. Taking or being subjected to treatments or therapies approved by a government department for curing a specific disease (e.g., a type of cancer, skin disease, or kidney disease);
19. Exercising or participating a religious activity, such as praying for peace or worshiping God;
20. Being exposed directly or indirectly to sunshine or sunlight (in the morning between, for example, 10 minutes before sunrise and 50 minutes after sunrise (containing significant amount of infrared (IR) light); or around noon, for example, between 11:30 AM to 12:30 PM (containing significant amount of ultra-violet (UV) light); or in the afternoon, for example, between 50 minutes before sunset and 10 minutes after sunset (containing significant amount of infrared (IR) light));
21. Being exposed to the lamp light or the light emitting diode (LED) light, which may include a whole spectrum of visible lights, IR light, red light, green light, blue light, or UV light, or a combination of more than one of the above lights;
22. Exercising or being subjected to programs, therapies, methods, apparatus and/or systems for improving body's self-healing, for example, a method or therapy (e.g., Hyperbaric oxygen therapy) performed after injury or surgery for improving self-healing;
23. Drinking coffee such as black coffee;
24. Drinking tea such green tea, black tea, or jasmine tea;
25. Drinking red wine;
26. Taking melatonin;
27. Listening to music such as Mozart's or Beethoven's symphony;
28. Injecting a substance (e.g., a nutrient or supplement) containing fucoidan or oligo fucoidan;
29. Taking hormone supplements or being subjected to a hormone injection;
30. Injecting a granulocyte-colony stimulating factor (G-CSF or GCSF), which is a cytokine or hormone (glycoprotein) that can stimulate the bone marrow to produce granulocytes and stem cells and release them into the bloodstream;
31. Being subjected to a course of GCSF injections; and
32. Taking a nutrient, a nutrient product, a nutrient fluid, a nutrient drink, a nutrient liquid, or a nutrient food containing (1) varieties of amino acids (such as Arginine, Histidine, Lysine, Aspartic acid, Glutamic acid, Serine, Threonine, Asparagine, Glutamine, Cysteine, Valine, Proline, Glycine, Selenocysteine, Alanine, Isoleucine, Leucine, Phenylalanine, Methionine, Tyrosine, or Tryptophan), (2) balanced amino acids, or (3) 9 essential amino acids (i.e., Histidine, Isoleucine, Leucine, Lysine, Methionine, Phenylalanine, Threonine, Tryptophan and Valine) for human bodies. For examples: (a) Product produced or extracted from the fermentation of red, green, black beans; (b) Liquid, fluid, or drink produced from fermentation of a fruit or a combination of fruits, such as sugar beet, apple, guava, kiwi, grape, pineapple, red pitaya (dragon fruit), green papaya, tomato, and/or avocado, etc.; (c) A medicinal liquor (or called medicinal wine, medicated liquor, or medicated wine) made from, e.g., immersing one Chinese medicine or multiple Chinese medicines in liquor or wine for a period of time, such as ginseng wine made from immersing ginseng in a high alcohol concentration rice wine for a month.

### Embodiments:

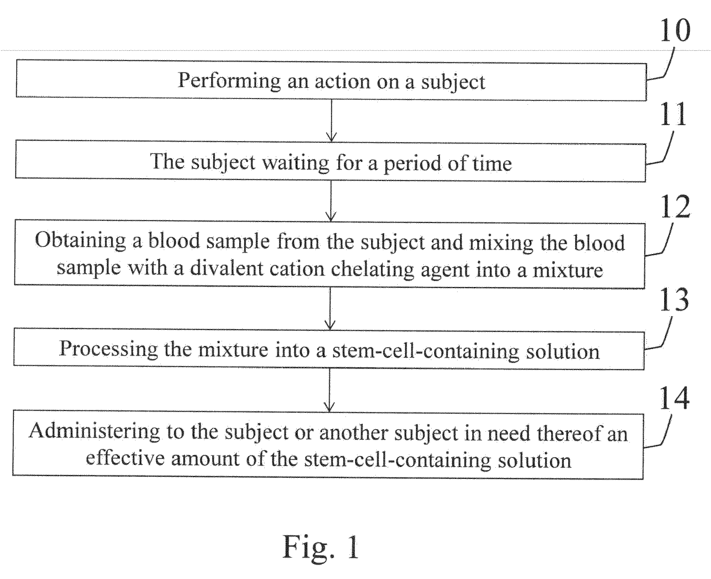

Fig. 1 is a flow chart illustrating a treatment or therapy for an autoimmune disease, such as ankylosing spondylitis, rheumatoid arthritis, or transient acantholytic dermatosis, according to an embodiment of the present disclosure. Referring to Fig. 1, in a step 10, a subject takes or is subjected to an action, which may be one of the above-mentioned actions (X). The subject, for example, is a human (e.g., child, teenager, adult, or graybeard) or an animal (e.g., mammal). Examples of the animal are listed as below: primate (e.g., monkey or gorilla), dog, rodent (e.g., mouse or guinea pig), cat, horse, cow, cattle, sheep, pig, chicken, duck, goose, bird, and elephant.

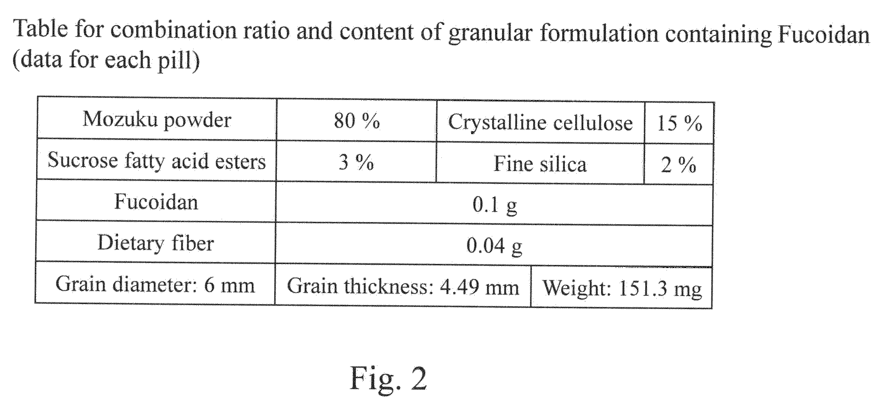

For example, in the step 10, the subject takes or ingests a stem-cell mobilization agent such as a fucoidan-containing compound. The fucoidan-containing compound may be a brown algae supplement. Fig. 2 shows the content/ingredient information of the brown algae supplement. In Fig. 2, it shows that a pill of the brown algae supplement contains 80% of a mozuku powder, 15% of crystalline cellulose, 3% of sucrose fatty acid esters, and 2% of micro or fine silica (containing silicon dioxide). The mozuku powder may be extracted from mozuku brown algae (one kind of seaweed) grown in the sea around and near Okinawa, Japan. The mozuku powder is then mixed with crystalline cellulose, sucrose fatty acid esters, and micro or fine silica (containing silicon dioxide) to form the pill of the brown algae supplement, which contains 0.1 grams of fucoidan. In the step 10, the subject may take or ingest 20 or more pills (e.g., at least 30 pills) of the brown algae supplement, which means the subject may take or ingest 2 grams or more (such as at least 3 grams) of fucoidan. In another example, the subject may be injected with a granulocyte-colony stimulating factor (GCSF), which is a stem-cell mobilization agent, or may be subjected to a course of GCSF injections.

In a step 11 of Fig. 1, after the action mentioned in the step 10 is performed on the subject, the subject waits for a (predetermined) period of time, such as between 15 minutes and 60 minutes, between 20 minutes and 100 minutes, between 30 minutes and 4 hours, between 60 minutes and 90 minutes, between 0.5 hours and 3 hours, between 1 hour and 6 hours, between 1 hour and 12 hours, between 12 hours and 36 hours, or between 36 hours and 50 hours. With the steps 10 and 11, one or more specific types of somatic stem cells, such as SB cells, can be mobilized into the subject's peripheral blood from, e.g., the subject's bone marrow; therefore, the peripheral blood of the subject becomes enriched with the one or more specific types of somatic stem cells. The one or more specific types of somatic stem cells, for example, may be or may include one or more types of stem cells described in the section of the "Description of stem cells." For instance, the one or more specific types of somatic stem cells may be or may include somatic stem cells less than 6 micrometers in size, and more preferably greater than 1 or 2 micrometers in size, such as CD349(+) somatic stem cells (i.e., SB-1 cells) and/or Lgr5(+) somatic stem cells (i.e., SB-2 cells). In another example, the one or more specific types of somatic stem cells may be or may include somatic stem cells having a size between 1 and 6 micrometers, such as SB-1 cells, SB-2 cells, BLSCs, VSELs, MSCs, and/or HSCs.

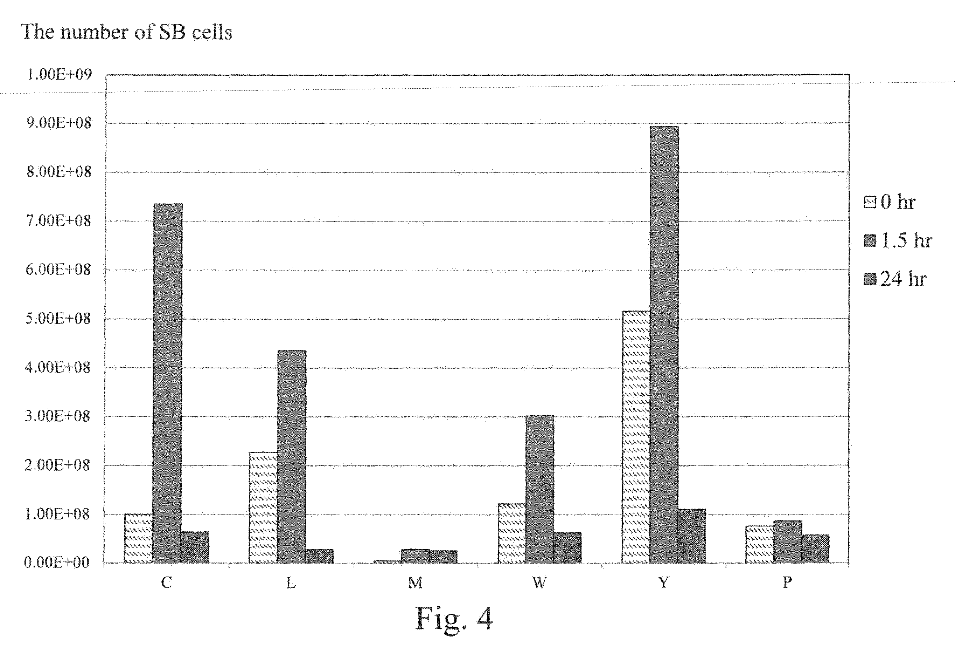

Fig. 4 is a graph presenting SB-cell data of six human subjects C, L, M, W, Y, and P obtained from peripheral-blood samples of the human subjects C, L, M, W, Y, and P in an experiment. In this experiment, the five human subjects C, L, M, W, and Y each orally ingested 20 pills of the brown algae supplement described in Fig. 2. In other words, each of the human subjects C, L, M, W, and Y orally ingested at least 2 grams of fucoidan. The human subject P was selected as a control in the experiment and did not ingest the brown algae supplement. In Fig. 4, 0 hr represents "before the ingestion of fucoidan" for the human subjects C, L, M, W, and Y and also represents "at the beginning of a control test" for the human subject P; 1.5 hr represents "at 1.5 hours after the ingestion of fucoidan" for the human subjects C, L, M, W, and Y and also represents "at 1.5 hours after the beginning of the control test" for the human subject P; 24 hr represents "at 24 hours after the ingestion of fucoidan" for the human subjects C, L, M, W, and Y and also represents "at 24 hours after the beginning of the control test" for the human subject P. As shown in Fig. 4, a significant increase in the number of CD349(+) cells and Lgr5(+) cells (i.e., SB cells) was found at 1.5 hours after the ingestion of fucoidan, but an insignificant increase in the number of CD349(+) cells and Lgr5(+) cells was found at 1.5 hours after the beginning of the control test. Therefore, ingesting fucoidan, e.g., the brown algae supplement, can mobilize SB cells (e.g., CD349(+) cells and Lgr5(+) cells) into the peripheral blood or the bloodstream and enrich the peripheral blood or the bloodstream with SB cells.

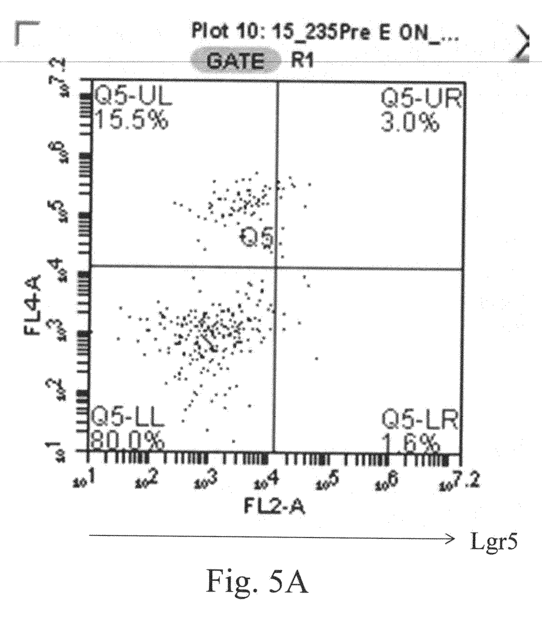

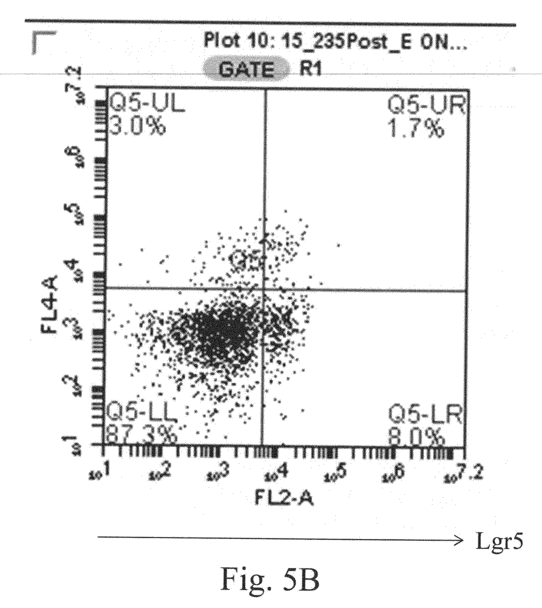

Fig. 5A shows flow cytometry data related to Lgr5(+) cells, i.e., SB-2 cells, in a first peripheral-blood sample obtained from a human subject before a course of GCSF injections at a dose of 5 micrograms/kg/day for 5 consecutive days. Fig. 5B shows flow cytometry data related to Lgr5(+) cells in a second peripheral-blood sample obtained from the human subject after the course of GCSF injections. In this experiment, the human subject was subjected to 5 micrograms/kg per day of GCSF as a single injection for five consecutive days. The second peripheral-blood sample was collected from the human subject at approximately 3.5 hours after last injecting GCSF. The results or data of Figs. 5A and 5B were obtained from a flow cytometer. The black points in the regions Q5-LR of Figs. 5A and 5B represent Lgr5(+) cells. With respect to the first peripheral-blood sample, the percentage of the number of Lgr5(+) cells in the region Q5-LR of Fig. 5A to the number of all particles or cells in the regions Q5-UL, Q5-UR, Q5-LL, and Q5-LR of Fig. 5A was 1.6%. With respect to the second peripheral-blood sample, the percentage of the number of Lgr5(+) cells in the region Q5-LR of Fig. 5B to the number of all particles or cells in the regions Q5-UL, Q5-UR, Q5-LL, and Q5-LR of Fig. 5B was 8%. Therefore, injecting GCSF can mobilize SB cells (especially Lgr5(+) cells) into the peripheral blood or the bloodstream and enrich the peripheral blood or the bloodstream with SB cells.

It can be seen from the above results or data that the action mentioned in the step 10, such as ingesting the brown algae supplement or receiving a course of GCSF injections, can cause the one or more specific types of somatic stem cells (e.g., SB cells) mobilized into the peripheral blood of the subject to increase at a time point, e.g., of 1.5 hours or between 1 hour and 6 hours after the subject having taken or been subjected to the action. Therefore, the steps 10 and 11 may be performed to enrich the one or more specific types of somatic stem cells, such as SB-1 cells and/or SB-2 cells, in the subject's peripheral blood.

Referring to Fig. 1, in a step 12, a blood sample is extracted, drawn, taken, obtained, collected or derived from the peripheral blood of the subject, a non-embryonic tissue of the subject, immediately after the step 11 into one or more first containers (such as a bag, one or more syringes, or one or more tubes) containing a divalent cation chelating agent, and mixed with the divalent cation chelating agent well so that a first mixture of the blood sample and the divalent cation chelating agent is formed in the one or more first containers. The divalent cation chelating agent, which is an anticoagulant, may be ethylenediaminetetraacetic acid (EDTA), such as K2 EDTA anticoagulant or K3 EDTA anticoagulant, having a weight, e.g., greater than 70 mg, such as between 90 and 900 mg, between 120 and 450 mg, or between 150 and 400 mg. Alternatively, the divalent cation chelating agent may be citrate having a weight, e.g., greater than 70 mg, such as between 90 and 900 mg, between 120 and 450 mg, or between 150 and 400 mg. The blood sample contains a plurality of cells, including small cells less than and equal to 6 micrometers in size and large cells greater than 6 micrometers in size. The small cells, for example, contain platelets (which, for example, are less than 6 micrometers in size) and small somatic stem cells (which are less than and equal to 6 micrometers in size, and more preferably greater than 1 or 2 micrometers in size). For instance, the small somatic stem cells contain the one or more specific types of somatic stem cells (e.g., SB cells), BLSCs (i.e., CD66e(+) somatic stem cells), VSELs (e.g., CD133(+) somatic stem cells and CD34(+) somatic stem cells), MSCs, and HSCs. The large cells, for example, contain large somatic stem cells greater than 6 micrometers in size and lineage cells containing red blood cells and white blood cells. The blood sample, for example, may have a volume greater than or equal to 20, 30 or 45 milliliters, such as between 20 and 250 milliliters, between 60 and 500 milliliters, between 80 and 250 milliliters or between 100 and 200 milliliters. To obtain or form the first mixture, the blood sample may be mixed with 1.5 mg or more, such as between 1.6 and 2.0 mg, of the divalent cation chelating agent (such as K2 EDTA, K3 EDTA, or citrate) per milliliter of the blood sample.

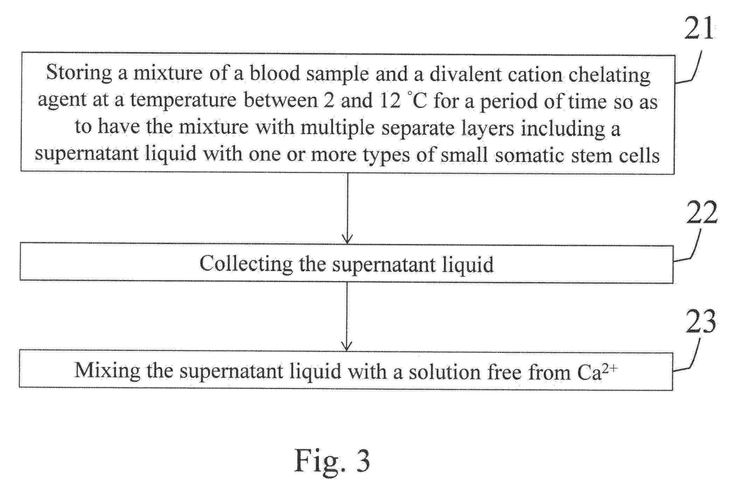

Next, in a step 13 of Fig. 1, steps 21 through 23 illustrated in Fig. 3 are performed on the first mixture to be processed into a stem-cell-containing solution (or called a stem-cell mixture). Referring to Fig. 3, in the step 21, the first mixture may be stored at a temperature between 2 degrees Celsius (°C) and 12 °C, and more preferably between 2 °C and 7 °C, or of about 4 °C, in a suitable facility (e.g., a refrigerator or other device used to keep things cold) for a predetermined period of time, such as between 3 hours and 72 hours, and more preferably between 3 hours and 6 hours, between 6 hours and 72 hours, between 6 hours and 48 hours, between 16 hours and 72 hours, between 16 hours and 48 hours, between 36 hours and 60 hours, between 48 hours and 72 hours, or around 48 hours. After the first mixture has been stored for the predetermined period of time, the one or more specific types of somatic stem cells (e.g., SB cells) in the first mixture are activated by the divalent cation chelating agent (such as K2 EDTA, K3 EDTA, or citrate), i.e., the cell cycle of the one or more specific types of somatic stem cells is activated from Go into G₁. The activation relates to the ability of the divalent cation chelating agent (such as K2 EDTA or K3 EDTA) to repress p53's function (presumably by chelating Zn²⁺), thereby allowing the one or more specific types of somatic stem cells to turn from the Go quiescence stage into the cell cycle G₁. As the p53 protein requires Zn²⁺ to fold properly and form a functional protein, chelating Zn²⁺ by the divalent cation chelating agent would be a key step to activate the one or more specific types of somatic stem cells. It is possible that the divalent cation chelating agent can chelate other divalent ions (e.g., Ca²⁺) and thereby activates the one or more specific types of somatic stem cells that are in Go phase.

After having been stored at the temperature for the predetermined period of time, the first mixture is formed with two or more separate layers (which, for example, include an upper layer and a lower layer) because of gravity. One of the separate layers (such as the upper layer or the topmost one of the separate layers), for example, is a supernatant liquid (which may have a volume between 20 and 250 milliliters, between 40 and 125 milliliters, or between 50 and 100 milliliters) and can be categorized into three parts - platelets (which, for example, are less than 6 micrometers in size), serum, and the small somatic stem cells containing, e.g., the one or more specific types of somatic stem cells (e.g., SB cells), BLSCs (i.e., CD66e(+) somatic stem cells), VSELs (e.g., CD133(+) somatic stem cells and CD34(+) somatic stem cells), MSCs, and HSCs. The supernatant liquid in said one of the separate layers may also include the divalent cation chelating agent (e.g., EDTA) and/or growth factors. Another one of the separate layers such as the lower layer includes an extremely large percent of the large cells containing the lineage cells (such as red and white blood cells) and the large somatic stem cells in the first mixture. For example, said another one of the separate layers may have greater than 95, 98, or 99 percent of the large cells in the first mixture. Accordingly, the supernatant liquid substantially excludes the large cells (such as red blood cells and white blood cells) or may include less than 0.5, 1, 2, or 5 percent of the large cells in the first mixture. The supernatant liquid, for example, may include less than 1 or 2 percent of red blood cells in the first mixture and less than 1 or 2 percent of white blood cells in the first mixture; at least 98 or 99 percent of red blood cells in the first mixture and at least 98 or 99 percent of white blood cells in the first mixture may gather in said another one of the separate layers (e.g., the lower layer). The ratio value of the volume of the supernatant liquid to the volume of the blood sample or the first mixture may range from one third to one half. In summary, in accordance with the steps 12 and 21, the blood sample from the subject is incubated with the divalent cation chelating agent at the temperature for the predetermined period of time so that the blood sample is formed with the separate layers including the supernatant liquid.

Next, in the step 22, the vast majority of or substantially all of the supernatant liquid are collected or transferred into a second container, such as a bag, a glass bottle, or a syringe. The supernatant liquid in the second container (hereinafter the "second mixture") contains the small cells, such as platelets and the small somatic stem cells containing, e.g., the one or more specific types of somatic stem cells (e.g., SB cells), BLSCs (i.e., CD66e(+) somatic stem cells), VSELs (e.g., CD133(+) somatic stem cells and CD34(+) somatic stem cells), MSCs, and HSCs. The number of somatic stem cells greater than 2 micrometers and less than 6 micrometers in size in the second mixture, for example, is greater than or equal to 10 million or 30 million, and more preferably between 25 million and 300 million, between 30 million and 500 million, or between 10 million and 500 million. The second mixture may also contain the divalent cation chelating agent (e.g., EDTA) and/or growth factors. The percentage of the number of the large cells in the second mixture to the number of total particles or cells in the second mixture may be less than 5%, 1%, or 0.5%, and more preferably less than 0.1% or 0.01%; the percentage of the number of the small cells in the second mixture to the number of the total particles or cells in the second mixture may be greater than 95%, 99%, or 99.5%, and more preferably greater than 99.9% or 99.99%.

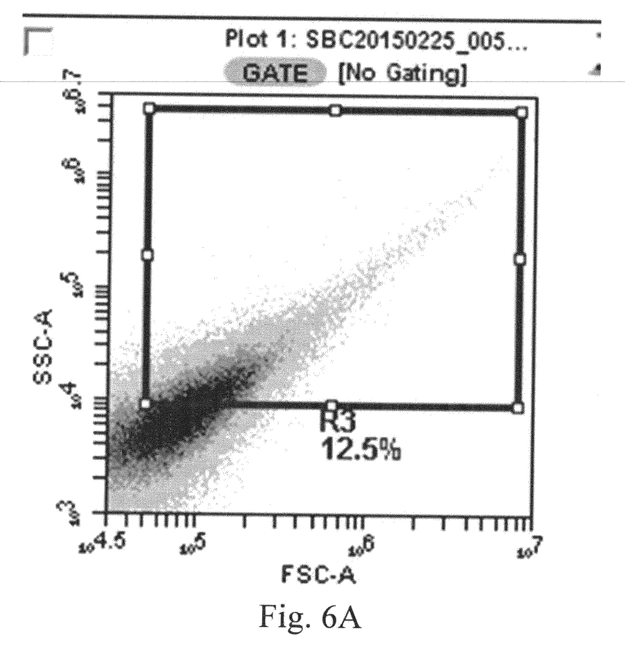

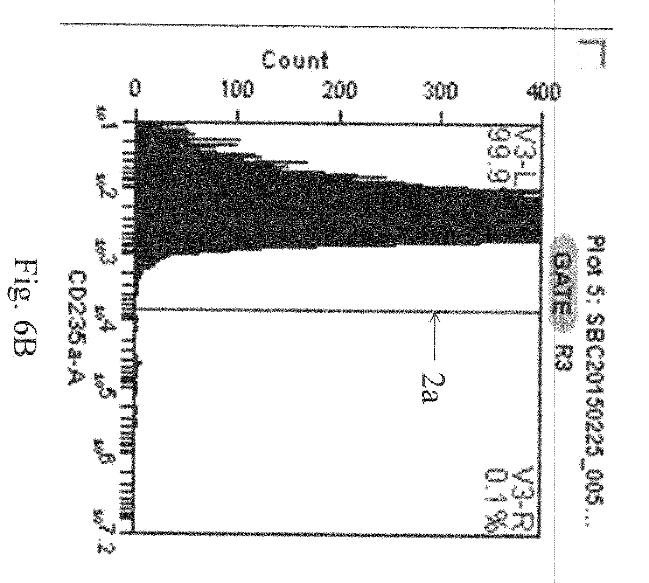

The number of red blood cells per milliliter of the second mixture is calculated based on, e.g., data of Figs. 6A and 6B. Fig. 6A is obtained by analyzing 10 microliters (µl) of the second mixture with a flow cytometer. Red blood cells (i.e., CD235a(+) cells) are in a region R3 of Fig. 6A; the number of all cells in the region R3 of Fig. 6A is 25000. Fig. 6B shows the distribution of CD235a fluorescence intensity in all cells in the region R3 of Fig. 6A. In Fig. 6B, the fluorescence histogram is separated into a region V3-L with low fluorescence intensity and a region V3-R with high fluorescence intensity by a vertical line 2a (i.e., reference). The region V3-R of Fig. 6B represents cells stained positive for CD235a, i.e., red blood cells; the region V3-L of Fig. 6B represents cells stained negative for CD235a. The result of Fig. 6B indicates that the percentage of the number of red blood cells in the region R3 of Fig. 6A to the number of all cells in the region R3 of Fig. 6A is 0.1%. By multiplying the number of all cells in the region R3 of Fig. 6A, i.e., 25000, by the percentage of the number of red blood cells in the region R3 of Fig. 6A to the number of all cells in the region R3 of Fig. 6A, i.e., 0.1%, the number of red blood cells in the 10 microliters of the second mixture is calculated and found to be equal to 25. Therefore, the number of red blood cells per milliliter of the second mixture is calculated from the number of red blood cells in the 10 microliters of the second mixture and found to be equal to 2500. It can be seen from the above result that the number of red blood cells per milliliter of the second mixture can be less than 10⁵ or 10⁴. Preferably, the number of red blood cells per milliliter of the second mixture is less than 10³. In addition, the percentage of red blood cells in cells greater than 2 micrometers and less than 10 micrometers in size in the second mixture may be less than 3%, and more preferably less than 2%, 1%, or 0.5%.

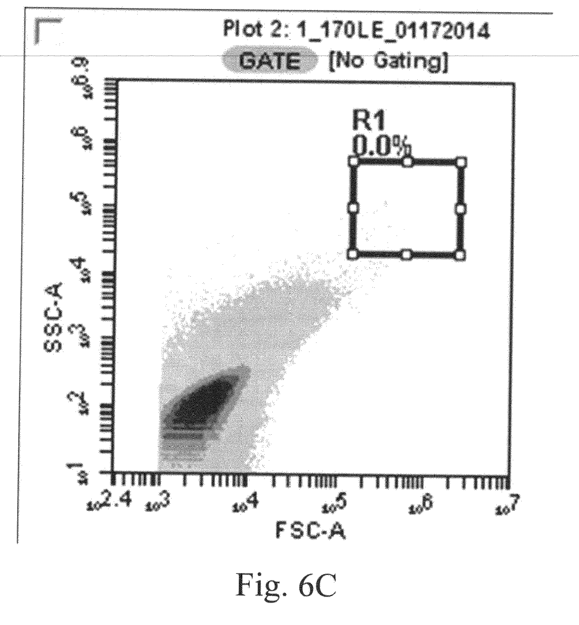

The number of white blood cells per milliliter of the second mixture is calculated based on, e.g., flow cytometry data in Fig. 6C. The flow cytometry data in Fig. 6C are obtained by analyzing 50 microliters (µl) of the second mixture with a flow cytometer. In Fig. 6C, a region R1 is white-blood-cell (WBC) gating. Based on the flow cytometry data in Fig. 6C, the number of white blood cells in the 50 microliters of the second mixture is calculated and found to be equal to 30. Therefore, the number of white blood cells per milliliter of the second mixture is calculated from the number of white blood cells in the 50 microliters of the second mixture and found to be equal to 600. It can be seen from the above result that the number of white blood cells per milliliter of the second mixture can be less than 10⁴ or 10³. Preferably, the number of white blood cells per milliliter of the second mixture is less than 10². In addition, the percentage of white blood cells in cells greater than 2 micrometers and less than 20 micrometers in size in the second mixture may be less than 2%, and more preferably less than 1%, 0.5%, or 0.1%.

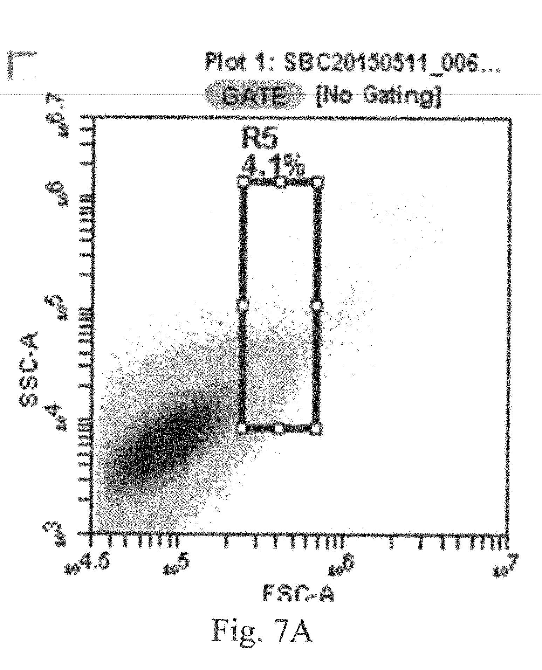

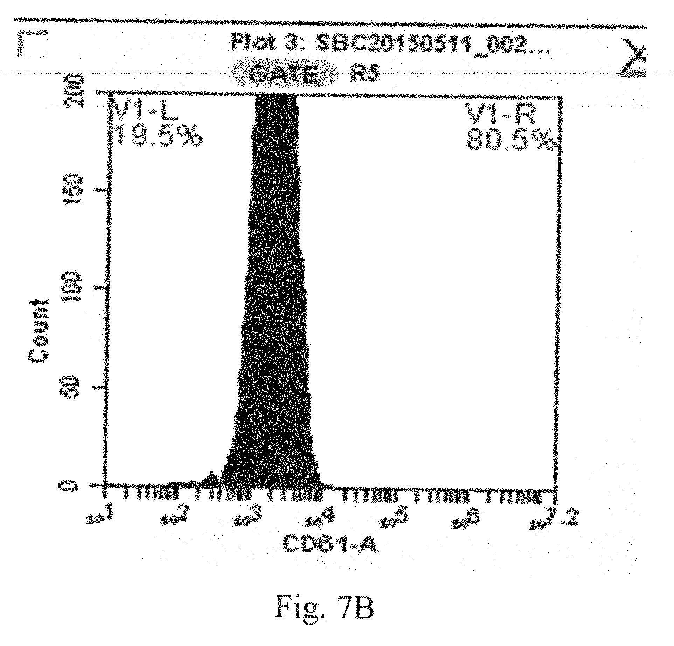

Fig. 7A shows a forward scattering (FSC) versus side scattering (SSC) flow cytometry dot plot, which is obtained by analyzing 5.6 microliters (µl) of the second mixture with a flow cytometer. In Fig. 7A, a region R5 represents cells that are less than 6 micrometers and greater than 2 micrometers in size. In other words, all cells in the region R5 are greater than 2 micrometers and less than 6 micrometers in size. Fig. 7B shows the distribution of CD61 fluorescence intensity in all cells in the region R5 of Fig. 7A. In Fig. 7B, the fluorescence histogram is separated into a region V1-L with low fluorescence intensity and a region V1-R with high fluorescence intensity by a vertical line (i.e., reference). The region V1-R of Fig. 7B represents cells stained positive for CD61, i.e., platelets; the region V1-L of Fig. 7B represents cells stained negative for CD61. The result of Fig. 7B indicates that the percentage of the number of platelets in the region R5 to the number of all cells in the region R5 is 80.5%. Therefore, the percentage of platelets in cells less than 6 micrometers and greater than 2 micrometers in size in the second mixture may be greater than 75% or 80%, and more preferably between 75% and 85%.

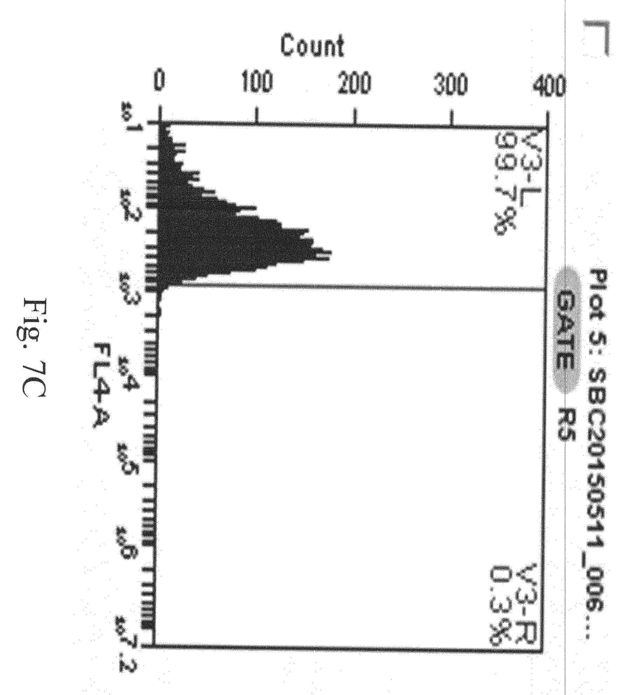

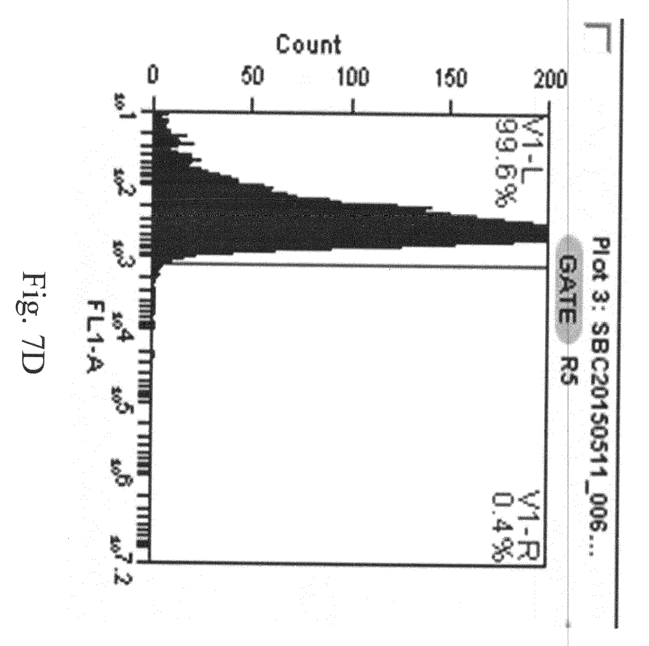

Fig. 7C shows the distribution of CD 133 fluorescence intensity in all cells in the region R5 of Fig. 7A. In Fig. 7C, the fluorescence histogram is separated into a region V3-L with low fluorescence intensity and a region V3-R with high fluorescence intensity by a vertical line (i.e., reference). The region V3-R of Fig. 7C represents cells stained positive for CD133, i.e., VSELs; the region V3-L of Fig. 7C represents cells stained negative for CD133. The result of Fig. 7C indicates that the percentage of the number of CD133(+) cells in the region R5 to the number of all cells in the region R5 is 0.3%. It can be seen from the result that less than 1% of cells in the second mixture express CD133. Fig. 7D shows the distribution of CD34 fluorescence intensity in all cells in the region R5 of Fig. 7A. In Fig. 7D, the fluorescence histogram is separated into a region V1-L with low fluorescence intensity and a region V1-R with high fluorescence intensity by a vertical line (i.e., reference). The region V1-R of Fig. 7D represents cells stained positive for CD34, i.e., VSELs; the region V1-L of Fig. 7D represents cells stained negative for CD34. The result of Fig. 7D indicates that the percentage of the number of CD34(+) cells in the region R5 to the number of all cells in the region R5 is 0.4%. Therefore, the percentage of the combination of CD133(+) cells and CD34(+) cells in cells less than 6 micrometers and greater than 2 micrometers in size in the second mixture may be less than 2%, and more preferably less than 1% or 0.5%.

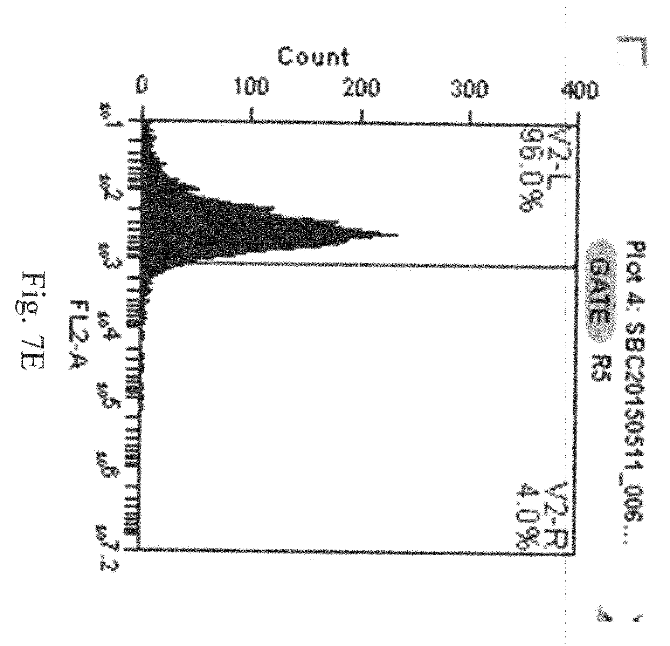

Fig. 7E shows the distribution of CD66e fluorescence intensity in all cells in the region R5 of Fig. 7A. In Fig. 7E, the fluorescence histogram is separated into a region V2-L with low fluorescence intensity and a region V2-R with high fluorescence intensity by a vertical line (i.e., reference). The region V2-R of Fig. 7E represents cells stained positive for CD66e, i.e., BLSCs; the region V2-L of Fig. 7E represents cells stained negative for CD66e. The result of Fig. 7E indicates that the percentage of the number of CD66e(+) cells in the region R5 to the number of all cells in the region R5 is 4%. Therefore, the percentage of CD66e(+) cells in cells less than 6 micrometers and greater than 2 micrometers in size in the second mixture may be less than 6%, and more preferably less than 5% or 4.5%.

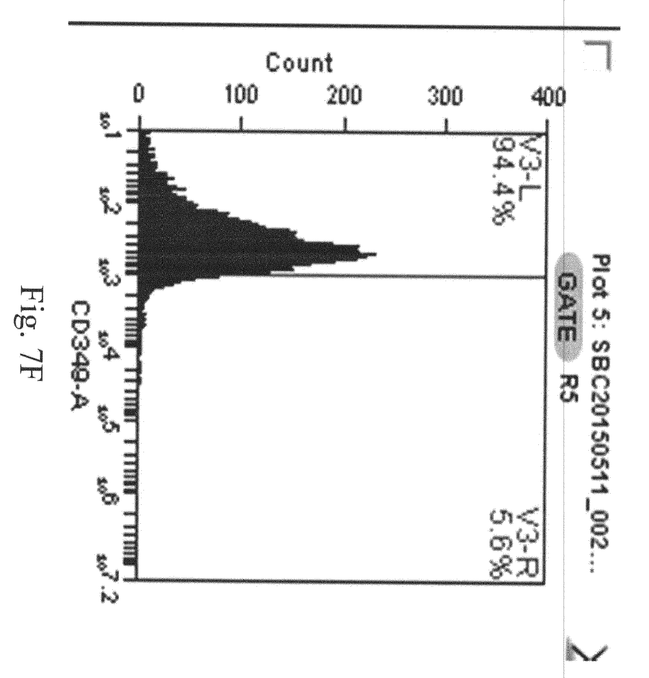

Fig. 7F shows the distribution of CD349 fluorescence intensity in all cells in the region R5 of Fig. 7A. In Fig. 7F, the fluorescence histogram is separated into a region V3-L with low fluorescence intensity and a region V3-R with high fluorescence intensity by a vertical line (i.e., reference). The region V3-R of Fig. 7F represents cells stained positive for CD349, i.e., SB-1 cells; the region V3-L of Fig. 7F represents cells stained negative for CD349. The result of Fig. 7F indicates that the percentage of the number of CD349(+) cells in the region R5 to the number of all cells in the region R5 is 5.6%. Therefore, the percentage of CD349(+) cells in cells less than 6 micrometers and greater than 2 micrometers in size in the second mixture may be greater than 4% or 5%, and more preferably between 4.5% and 10%.

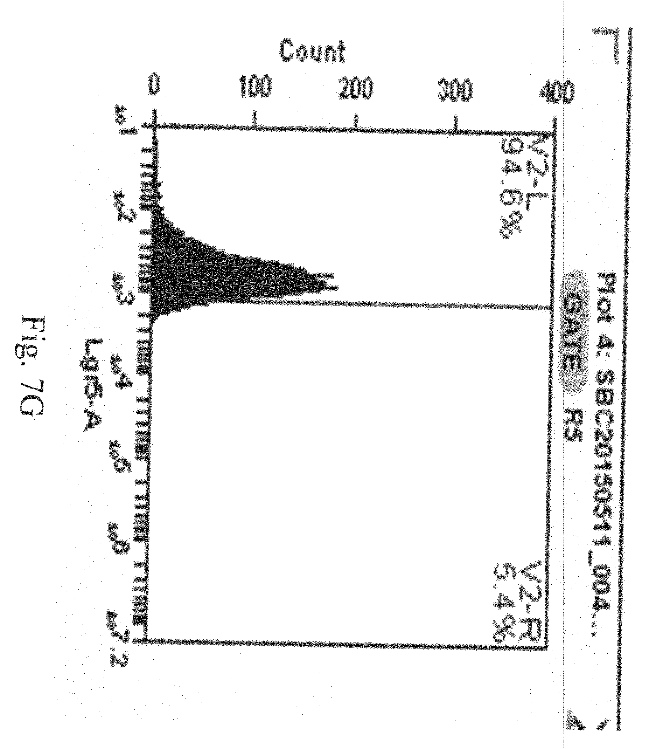

Fig. 7G shows the distribution of Lgr5 fluorescence intensity in all cells in the region R5 of Fig. 7A. In Fig. 7G, the fluorescence histogram is separated into a region V2-L with low fluorescence intensity and a region V2-R with high fluorescence intensity by a vertical line (i.e., reference). The region V2-R of Fig. 7G represents cells stained positive for Lgr5, i.e., SB-2 cells; the region V2-L of Fig. 7G represents cells stained negative for Lgr5. The result of Fig. 7G indicates that the percentage of the number of Lgr5(+) cells in the region R5 to the number of all cells in the region R5 is 5.4%. Therefore, the percentage of Lgr5(+) cells in cells less than 6 micrometers and greater than 2 micrometers in size in the second mixture may be greater than 4% or 5%, and more preferably between 4.5% and 10%.

In the embodiment, the step 22 may be deemed as a method for stem cell purification/isolation. The term "purification/isolation" means the act of substantially separating small cells described in the step 12 (e.g., cells greater than 2 micrometers and less than 6 micrometers in size) in a mixture or blood sample from large cells described in the step 12 (e.g., cells greater than 6 micrometers in size) in the mixture or blood sample, i.e., the act of obtaining the small cells in the mixture or blood sample by removing most or substantially all of the large cells in the mixture or blood sample.

Next, in the step 23, the second mixture (i.e., the supernatant liquid in the second container) may be transferred to be mixed with a medium or solution free from Ca²⁺ having a volume, e.g., greater than 400 milliliters, such as between 500 and 900 milliliters, into the stem-cell-containing solution, which thereby contains the small cells, such as platelets and the small somatic stem cells containing, e.g., the one or more specific types of somatic stem cells (e.g., SB cells), BLSCs (i.e., CD66e(+) somatic stem cells), VSELs (e.g., CD133(+) somatic stem cells and CD34(+) somatic stem cells), MSCs, and HSCs. The medium or solution free from Ca²⁺, such as a salt solution, may be further free from any other divalent ions, including Mg²⁺. The salt solution may be a NaCl-containing solution, such as normal saline. Normal saline is the commonly used phrase for a solution of 0.90% w/v of NaCl, about 300 mOsm/L or 9.0 g per liter. Alternatively, the stem-cell-containing solution may be further passed through a filter (such as a 170-micrometer filter) in order that impurities are filtered from the stem-cell-containing solution. The filtered stem-cell-containing solution still contains the small cells, such as platelets and the small somatic stem cells including, e.g., the one or more specific types of somatic stem cells (e.g., SB cells), BLSCs (i.e., CD66e(+) somatic stem cells), VSELs (e.g., CD133(+) somatic stem cells and CD34(+) somatic stem cells), MSCs, and HSCs.

According to the results from Figs. 6A-6C and 7A-7G, it can be estimated, assumed, presumed or obtained that the percentage of SB-1 cells in cells greater than 2 micrometers and less than 6 micrometers in size in the (filtered) stem-cell-containing solution may be greater than 4% or 5%, and more preferably between 4.5% and 10%; the percentage of SB-2 cells in cells greater than 2 micrometers and less than 6 micrometers in size in the (filtered) stem-cell-containing solution may be greater than 4% or 5%, and more preferably between 4.5% and 10%; the percentage of BLSCs in cells less than 6 micrometers and greater than 2 micrometers in size in the (filtered) stem-cell-containing solution may be less than 6%, and more preferably less than 5% or 4.5%; the percentage of VSELs in cells less than 6 micrometers and greater than 2 micrometers in size in the (filtered) stem-cell-containing solution may be less than 2%, and more preferably less than 1% or 0.5%; less than 1% of cells in the (filtered) stem-cell-containing solution express CD133; the percentage of platelets in cells less than 6 micrometers and greater than 2 micrometers in size in the (filtered) stem-cell-containing solution may be greater than 75% or 80%, and more preferably between 75% and 85%; the number of white blood cells per milliliter of the (filtered) stem-cell-containing solution may be less than 10⁴ or 10³, and more preferably less than 10²; the percentage of white blood cells in cells greater than 2 micrometers and less than 20 micrometers in size in the (filtered) stem-cell-containing solution may be less than 2%, and more preferably less than 1%, 0.5% or 0.1%; the number of red blood cells per milliliter of the (filtered) stem-cell-containing solution may be less than 10⁵ or 10⁴, and more preferably less than 10³; the percentage of red blood cells in cells greater than 2 micrometers and less than 10 micrometers in size in the (filtered) stem-cell-containing solution may be less than 3%, and more preferably less than 2%, 1%, or 0.5%. The (filtered) stem-cell-containing solution, for example, may further contain the divalent cation chelating agent (e.g., EDTA), growth factors, and/or a salt (such as sodium chloride).

Referring to Fig.1, in a step 14, an effective amount of the (filtered) stem-cell-containing solution is injected intravenously into the subject (i.e., intravenous injection of autologous somatic stem cells) or another subject (i.e., intravenous injection of allogenic somatic stem cells) for treating an autoimmune disease, such as ankylosing spondylitis, rheumatoid arthritis, or transient acantholytic dermatosis (i.e., Grover's disease), in the subject or the other subject. In other words, an effective amount of the (filtered) stem-cell-containing solution is administered to the subject (e.g., by intravenous injection of autologous somatic stem cells) or the other subject (e.g., by intravenous injection of allogenic somatic stem cells) for treating the autoimmune disease in the subject or the other subject. The other subject may be a species in the same biological category as the subject and may have the same blood type as the subject. The term "autologous somatic stem cell" refers to a somatic stem cell that is genetically the individual's own. The term "allogenic somatic stem cell" refers to a stem cell from another individual of the same species (genetically different). An "effective amount" refers to an amount of a composition, agent or substance that is capable of producing a (medically) desirable result in a treated subject, wherein the composition, agent or substance may be the (filtered) stem-cell-containing solution. "Treating" refers to administration of a composition, agent or substance such as the (filtered) stem-cell-containing solution to a subject, who is suffering from or is at risk for developing a disease or disorder (e.g., an autoimmune disease), with the purpose to cure, inhibit, alleviate, relieve, remedy, delay the onset of, prevent, or ameliorate the disease or disorder, the symptom of the disease or disorder, the disease state secondary to the disorder or disorder, or the predisposition toward the damage/disorder. Alternatively, the steps 10 and 11 may be omitted, and the steps 12, 13 and 14 may be performed to obtain or prepare the (filtered) stem-cell-containing solution to be injected intravenously into the subject or the other subject for treating the autoimmune disease.

In an alternative embodiment, the second mixture (i.e., the supernatant liquid in the second container), which is not mixed with anything (e.g., the medium or solution free from Ca²⁺), may be injected intravenously into the subject (i.e., intravenous injection of autologous somatic stem cells) or the other subject (i.e., intravenous injection of allogenic somatic stem cells) for treating the autoimmune disease. In other words, an effective amount of the second mixture is administered to the subject (e.g., by intravenous injection of autologous somatic stem cells) or the other subject (e.g., by intravenous injection of allogenic somatic stem cells) for treating the autoimmune disease in the subject or the other subject. In the embodiment, the second mixture is deemed as a stem-cell-containing solution (or called a stem-cell mixture). That is, in this embodiment, the stem-cell-containing solution is prepared by the steps 10-12, 21 and 22 or by the steps 12, 21 and 22.

Ankylosing spondylitis (AS) is an inflammatory, autoimmune disease or disorder. It mainly affects the axial skeleton, sacroiliac joints of the pelvis, and thoracic cage. As the disease progresses, patients with ankylosing spondylitis experience pain, joint stiffness, and the eventual loss of spinal mobility. Millions of people are affected by ankylosing spondylitis. Thus, there is a need for a safe and effective treatment for ankylosing spondylitis.

In the present disclosure, the (filtered) stem-cell-containing solution or the second mixture may be used as an injection liquid or solution for intravenous use or infusion. The injection liquid or solution can be used for relieving, inhibiting, treating or curing an autoimmune disease or disorder, such as arthritis or transient acantholytic dermatosis (i.e., Grover's disease), in an individual, such as a human. Examples of arthritis are rheumatoid arthritis, psoriatic arthritis, and ankylosing spondylitis.

### Experimental studies:

In a first experimental study, a patient having pain from ankylosing spondylitis received the treatment or therapy illustrated in Fig. 1. After receiving the treatment or therapy, the patient has not felt the pain from ribs due to ankylosing spondylitis more than four months. Accordingly, the treatment or therapy illustrated in Fig. 1 can be effective at relieving, inhibiting, treating or curing the pain from ribs due to ankylosing spondylitis.

In a second experimental study, a patient with transient acantholytic dermatosis, who had erythematous papules on the body, received the treatment or therapy illustrated in Fig. 1. It turned out that the erythematous papules disappear from the patient's body. Accordingly, the treatment or therapy illustrated in Fig. 1 can be effective at relieving, inhibiting, treating or curing the symptom from transient acantholytic dermatosis.

In a third experimental study, a patient (female, 26 years old) with ankylosing spondylitis and psoriatic arthritis received the treatment or therapy illustrated in Fig. 1. After receiving the treatment or therapy, she has felt that her health has improved for the better. She is able to do more of the everyday activities with ease. Several areas of her body that suffer from psoriasis have improved very much. She claims to have a lot more energy and has an average of 6 hours of sleep as compared to the time prior to the treatment or therapy when she only had 3 hours average. Her recovery time from her flare-ups, which are moments that cause her joints to become immobile, has decreased from 4-5 days to 24 hours after receiving the treatment or therapy.

In a fourth experimental study, a patient (female, 70 years old) with fibromyalgia, arthritis, and interstitial lung disease received the treatment or therapy illustrated in Fig. 1. After receiving the treatment or therapy, the patient has a lot more mobility in her knees as compared to before. Since being diagnosed with the interstitial lung disease in 2012, her last visit to the doctor has shown the "cloudiness" in her lungs has decreased by 10%. Her breathalyzer test received the best results since her diagnosis in 2012.

In a fifth experimental study, a patient (male, 69 years old) with Grover's disease and inflammation in the joints received the treatment or therapy illustrated in Fig. 1. After he received the treatment or therapy, his skin disease, caused by Grover's disease, began to disappear which doctors have told him is incurable. Also, his vision has improved to the point where he does not use his reading glasses as much. Prior to the treatment or therapy, he could not walk up the stairs without any pain in his legs. After the treatment or therapy, the pain in his knees and left foot went away after a week. His first treatment went so well, and he and his wife were able to ski consecutively for 45 days after his first treatment. He came back for a second treatment because he injured his leg while skiing.

In a sixth experimental study, a patient (male, 33 years old) with ankylosing spondylitis and high blood pressure received the treatment or therapy illustrated in Fig. 1. After receiving the treatment or therapy, he is able to sleep a lot more and walk further than the usual prior to the treatment or therapy. His hand no longer cramps up periodically, and his inflammation has dropped significantly. His blood pressure is stabilizing in addition with the medication he is taking for it. Prior to the treatment or therapy, he would take anti-anxiety and pain killers to deal with the pain, but since the treatment or therapy, he has stopped taking them.

Therefore, the treatment or therapy illustrated in Fig. 1 can be used for relieving, inhibiting, treating or curing an autoimmune disease or disorder (e.g., ankylosing spondylitis, rheumatoid arthritis, psoriatic arthritis, or Grover's disease) in an individual, such as a human.

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

A number of embodiments have been described. Nevertheless, it will be understood that various modifications may be made without departing from the spirit and scope of the invention. Accordingly, other embodiments are within the scope of the following claims.

## Claims

1. A method of preparing an injection solution, comprising:
storing a first mixture of a blood sample and a divalent cation chelating agent at a temperature between 2 and 12 degrees Celsius for a time period of from 3 hours to 72 hours so as to have said first mixture with multiple separate layers, wherein a liquid in one of said separate layers contains multiple somatic stem cells having a size ranging from 1 micrometer to 6 micrometers; and
collecting said liquid.

2. The method of Claim 1, after said collecting said liquid, further comprising mixing said liquid with a solution free from Ca²⁺.

3. The method of Claim 2, wherein said solution free from Ca²⁺ comprises normal saline.

4. The method of Claim 2 or 3, after said mixing said liquid with said solution free from Ca²⁺ into a second mixture, further comprising filtering said second mixture of said liquid and said solution free from Ca²⁺.

5. The method of any one of Claims 1 to 4, wherein said temperature is between 2 and 7 degrees Celsius.

6. The method of any one of Claims 1 to 5, wherein said divalent cation chelating agent comprises ethylenediaminetetraacetic acid (EDTA).

7. The method of any one of Claims 1 to 6, wherein said somatic stem cells contain CD349(+) stem cells.

8. The method of any one of Claims 1 to 7, wherein said somatic stem cells contain Lgr5(+) stem cells.

9. The method of any one of Claims 1 to 8, wherein said liquid contains said divalent cation chelating agent.

10. The method of any one of Claims 1 to 9, wherein said blood sample is obtained from a subject immediately after ingesting fucoidan for a period of time between 30 minutes and 4 hours.

11. The method of any one of Claims 1 to 10, wherein said time period is from 16 hours to 72 hours.

12. The method of any one of Claims 1 to 11, wherein the percentage of white blood cells in cells greater than 2 micrometers and less than 20 micrometers in size in said liquid is less than 2%.

13. The method of any one of Claims 1 to 12, wherein the percentage of red blood cells in cells greater than 2 micrometers and less than 10 micrometers in size in said liquid is less than 3%.

14. The method of any one of Claims 1 to 13, wherein said injection solution is used for an autoimmune disease.

15. The method of any one of Claims 1 to 13, wherein said injection solution is used for ankylosing spondylitis.
